# EUROPEAN PATENT SPECIFICATION

(11) **EP 2 745 817 B1**
(45) Date of publication and mention of the grant of the patent: **24.08.2016**
(21) Application number: 11813524.3
(22) Date of filing: 06.12.2011
(51) Int. Cl.: A61F 7/02, A61F 7/00, A61B 5/01

(54) **DEVICE FOR CONTROLLING - EITHER INCREASING OR DECREASING - THE BODY TEMPERATURE OF A PATIENT**
VORRICHTUNG ZUR REGELUNG - ENTWEDER ERHÖHUNG ODER VERRINGERUNG - DER KÖRPERTEMPERATUR EINES PATIENTEN
DISPOSITIF DE COMMANDE UTILISÉ POUR AUGMENTER OU RÉDUIRE LA TEMPÉRATURE CORPORELLE D'UN PATIENT EN MÉDECINE

(30) Priority: 18.08.2011 CL 20302011
(43) Date of publication of application: 25.06.2014
(73) Proprietor: Rivas Tapia, Rodrigo, La Reina, Santiago (CL)
(72) Inventor: Rivas Tapia, Rodrigo, La Reina, Santiago (CL)
(74) Representative: Carpintero Lopez, Francisco
(86) International application number: PCT/CL2011/000073
(87) International publication number: WO 2013/023317

(56) References cited:
- WO-A1-01/17470
- WO-A2-02/01171
- WO-A2-2007/089293
- US-A1- 2003 078 638

## Description

*The present invention relates to a device* for *controlling, either by* rising or
lowering, the body temperature of a medical patient. Particularly, the invention is applied in the treatment of patients with hypothermia requiring rising body temperature in a controlled fashion, as well as lowering the patient's temperature in case of therapeutic hypothermia.

### BACKGROUND OF THE INVENTION

Human beings are homothermous animals, i.e. they are able to regulate their own temperature, which means that under normal physiological conditions they maintain a constant body temperature and within a very narrow range, between 36.6 +/- 0.38°C, despite large oscillations at room temperature. This temperature is maintained by a balance between heat production and loss.

Main heat production sources are: i) heat generation by biochemical processes that occur in cells of our body and, ii) heat generation by chemical reactions produced inside the muscles upon contraction. In turn, heat loss sources are: i) radiation, which is the way objects with a temperature above absolute zero lose heat, ii) conduction, which is when a body with a given temperature is in direct contact with a cooler body so that part of the heat thereof is transferred; iii) convection, is the transfer of heat from the body to the air or water particles which are in contact therewith, wherein these particles are heated when are in contact with the body surface and, subsequently, when they leave this surface, it is occupied by other cooler particles which in turn are heated, and so on, and iv) evaporation, corresponds to heat loss through sweating which evaporates from the skin causing heat loss.

There exists a need for body temperature control within the clinical setting.

In the case of patients in Intensive Care Units (ICU), they undergo long surgical procedures. Likewise, patients rescued from the accident zone, suffer from heat loss, altering the balance between heat production and loss, either because at ICU patient clothes are removed so that the skin is exposed to punctures, inspections or surgeries, and where the skin is washed with cold liquids, or because cold liquid injections are administered to patients. In the case of accident injured patients exposed to the environmental conditions and lying on cold places (pavement or floor) with their clothes wet with water or blood who are subsequently rescued, their clothes are removed in order to inspect them and to identify and assess the bleeding sites. Many are already cold when the rescue unit arrives. Both groups of patients lose heat by radiation, conduction and convection and together with the inability to move due to unconsciousness (caused by serious head traumas or by general anesthesia) they neither can generate heat by muscle contraction nor can keep warm in order to lose less heat by radiation. The natural consequence is hypothermia.

Accidental hypothermia is defined as body temperature below 35°C. However, regardless of achieving those levels of temperature, any degree of cooling may be detrimental for patients.

This body cooling involves a number of consequences. Coagulation alterations leading to an increased bleeding in patients; the central nervous system is affected resulting in somnolence and the immune system is also affected making patients susceptible to infections. Within the cardiovascular system, due to the intense reaction of the organism to keep and recover heat, arterial hypertension and tachycardia occur in patients with risk factors (elderly patients with a history of heart diseases), may result in a cardiac arrest.

On the other hand, under other conditions, such as the case of those patients suffering a cardiac arrest (with absence of brain blood flow) or asphyxia, the only efficacious measure to be taken in order to stop or limit the area of brain cell death caused by the lack of oxygen is that patients undergo a cooling process as soon as possible (during a period of less than 6 hours), specifically, to achieve a hypothermia state at a temperature of 32°C. This is known as therapeutic hypothermia.

By achieving this temperature in patients, and specifically in their brains, the subsequent consequences associated with this kind of accidents are milder than would otherwise have been the case. The mortality of patients suffering a cardiac arrest that undergo hypothermia is lower than that of those treated at normal temperature. Hypothermia is a global standard treatment tool for treating newborns suffering from neonatal asphyxia. It is easy to implement in them because they are small, have larger body surface areas exposed relative their weight from where the heat is lost (compared to adults) and they tend to cool rapidly in a spontaneous way. In turn, although the benefits of this measure are clear, the implementation thereof, (i.e., the methods for lowering temperature), are not clearly established in adults.

With regard to the ways used to modify a patient's body temperature, there exist devices designed to exchange heat with a patient by making use of the principle of convection. A particular type of convection is air convection, which allows cold and heat transfer through generation of air currents at a set temperature in a closed system that surrounds a patient. Through this technique, good results with regard to heat preservation and temperature rising under hypothermia conditions are obtained, but since large air-inflatable sheets made of light material located over the patient's skin exposed area are required, the body inspection which is vital in serious injured patients is obstructed. On the other hand, in patients that undergo major surgeries, wherein the abdomen, thoracic cavity, or plastic or aesthetic surgery reconstruction are involved and wherein hypothermia occurs as a consequence of the exposure of large areas of the body at room temperature, the use of this system for preventing or treating hypothermia is confined to the lower "available", extremities, however, they are not that effective for capturing and transferring heat towards the whole body. As a reflex form, the extremities exposed to room air experience a reduced blood flow towards the surface, i.e., skin, tips of the toes, etc., due to vasoconstriction phenomena to avoid heat loss. Therefore, by applying an air convection heater to the extremities; the blood being the vehicle that transports this change of temperature to the rest of the body; and, as a result of the cold, the amount of blood that circulates superficially in the extremity decreases, only the skin can be warmed, and the rise in temperature can not be transferred to the rest of the body, or if this is achieved, it occurs at a very low speed.

The devices that use air convection together with these methods, i.e., cool air ventilated mattresses located over the body are not effective to induce hypothermia.

There also exist systems that use water convections. For example, in heart surgery with extracorporeal circulation, a basic premise is to stop the heart and withdraw the blood for the period that the surgery thereon lasts, this surgery either being coronary artery surgery wherein arteries become blocked resulting in cardiac arrests or cardiac valve surgery wherein in certain diseases arteries become so damaged that they have to be changed. Heart is stopped, and blood is withdrawn from vena cava to the ECC machine wherein CO₂ is removed and replaced with oxygen to be returned to the aorta artery. As a consequence of this process, heat from blood is lost resulting in hypothermia.

In the case of a surgery with ECC, since the hart is stopped and does not receive any blood, the patient may suffer damages or may die if they do not receive enough oxygen. To overcome these problems, surgeons, along with heart stoppage, try to make sure that heart is cooled at temperatures ranging from 28 to 32°C so that the heart can survive around 2 hours without receiving oxygen flow. A water convection temperature exchanger is used to keep the patient at this temperature. Blood is transported from the patient through tubes made of synthetic plastic material to the ECC machine, which in turn comprises a closed container containing circulating water through which piping containing blood passes through without being in direct contact with water but experiencing temperature changes according to the circulating water temperature. Inside this closed container water is circulated at varying temperatures according to what is determined by the heat exchange controllers, it is the most efficient water convection temperature transfer system currently available, capable of cooling patients to 10°C if required. Once the procedure is completed, and in order to remove patient from ECC, the patient's temperature must be rised, from the temperature during surgery to the normal temperature of approximately 37°C.

This is the most effective method for warming or cooling patient but the usefulness thereof is limited to the field of cardiac surgery due to the degree of invasion for the patient which is not devoid of complication, including death.

There exist noninvasive devices (i.e., devices that do not penetrate patient's anatomical barriers) based on the same principle, which consist of a hollow mattress where the patient lies down; the mattress being made of plastic material within which cold or hot water is circulated but it is not effective for heat transfer in adults or children and it is only used to cool down newborns as a neonatal asphyxia recovery therapy.

### PREVIOUS ART

US20050574723 describes a device for rising temperature at a specific site by radiation. This device comprises layers of flexible material and insulating material. According to this document, the device can be in the form of a blanket and the use thereof in therapeutic settings is disclosed. However, the effect achieved corresponds only to a local temperature rise and the goal thereof is to reduce pain associated with a specific area. The present invention does not relate to patient's local temperature modification, it is used to rise or lower body temperature of a patient in a controlled manner for therapeutic purposes.

US20050248939 describes a system and method for producing therapeutic hypothermia. The system comprises a controller and several garments, wherein garments are connected to a controller (battery operated console). The controller supplies cold fluids to garments so that the body surface is cooled down by such garments. Specifically, the garments described in this document correspond to a kind of helmet capable of lowering temperature, preferably at the patient's head. Unlike this document, the present invention is capable of rising or lowering the patient's body temperature in a controlled manner and it is not limited to the patient's head temperature lowering. WO2001US22036 describes a medial device capable of removing a large amount of heat from a zone close to the patient's carotid arteries. An endotracheal device that can be used together or separately is also mentioned in this document. In particular, it allows the patient's head temperature lowering while the present invention is capable of controlling the patient's body temperature without limiting to an increment or reduction, and without limiting to a specific body area.

GB20070004465 describes a flexible sheet comprising ducts through which fluids circulate. Such ducts are connected to a temperature controller. Among different uses, the invention is mentioned as a blanket for warming up or cooling down a patient requiring body temperature local or general control. In contrast, a specific form of the blanket and the specific location thereof together with a temperature sensor capable of properly controlling the patient's temperature are considered in the present invention. US20060419186 describes a patient warming system. It comprises a covering that may cover the whole torso and neck as well as areas from other parts of the body. It is also mentioned that the system may be selectively activated per zones depending on the capacity of the body zone to capture heat. In contrast, the present invention corresponds to a cervical blanket wherein body temperature can be controlled either by increasing or reducing temperature based on the therapeutic requirements.

WO2010US0040 describes a whole body blanket that can be inflated with warmed air for patient warming. The structure of the blanket allows a selective activation so that specific parts of the body are exposed to heat. The present invention relates to a small blanket located at the patient's cervical zone and allows controlling of the patient's body temperature either by rising or lowering temperature, for example, in the case of therapeutic hypothermia.

To the best of the applicant knowledge, the aforementioned apparatuses, devices and methods of the previous art relate to the temperature rising, either locally or corporal, or to the temperature lowering, wherein documents focusing on lowering the patient's head temperature stand out. On the contrary, the present invention corresponds to a body temperature control system that can be used, for example, in cases of therapeutic hypothermia, wherein patient's temperature is lowered in the first place, and then when the clinical goal is achieved; the patient is helped to recover the appropriate body temperature. The control of body temperature and not only local application of cold and heat, sets us apart from the other prior art alternatives.

### BRIEF DESCRIPTION OF THE INVENTION

The present invention, as defined in independent claim 1 and its dependent claims, corresponds to a noninvasive system for rising or lowering body temperature of a subject in a controlled manner, wherein temperature rising or lowering are carried out according to a predetermined temperature and according to the temperature measured by a body temperature sensor.

The present disclosure also considers a method for controlling the body temperature of a
patient, either by rising or lowering temperature.

### BRIEF DESCRIPTION OF THE FIGURES

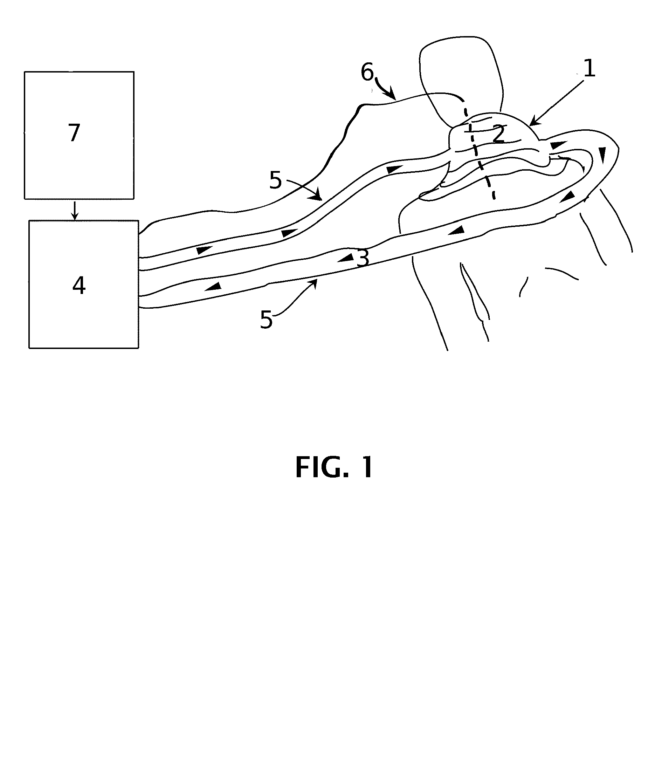

Figure 1 shows an schematic view of the system of the present invention comprising a cervical blanket (1) together with ducts (2) through which a fluid circulates (3); a fluid (3) capable of transferring heat from and to external bodies; a heat exchanger (4) which rises or lowers the temperature of the fluid circulating through the ducts (2) of the cervical blanket (1); means of connection (5) between the cervical blanket ducts and heat exchanger (4); a body temperature sensor (6); and a controller (7).

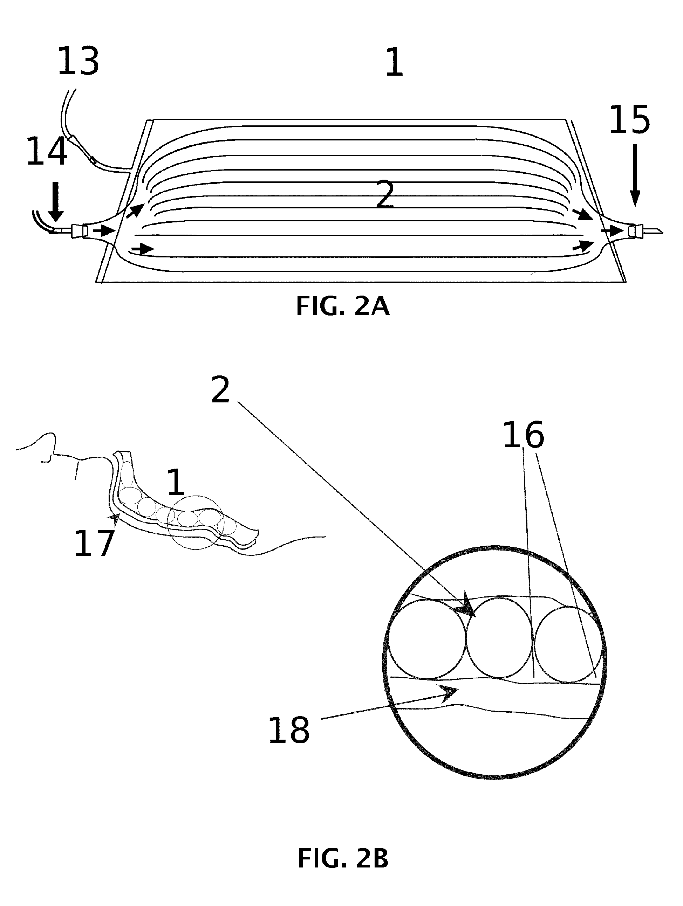

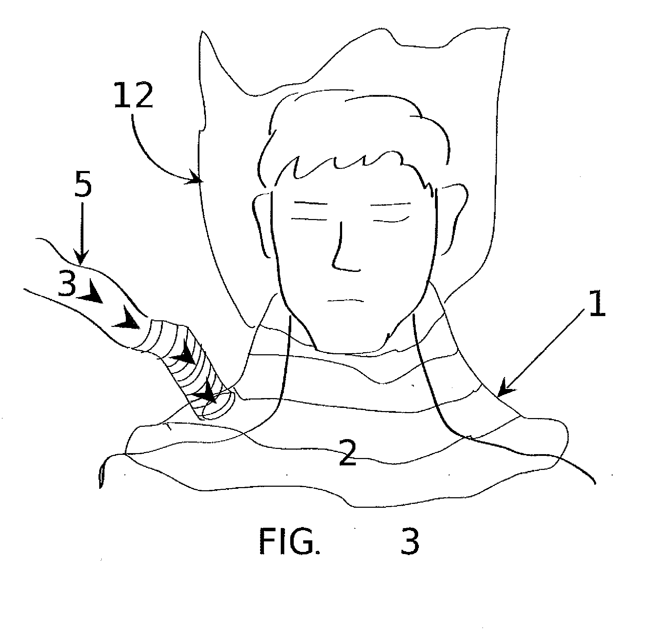

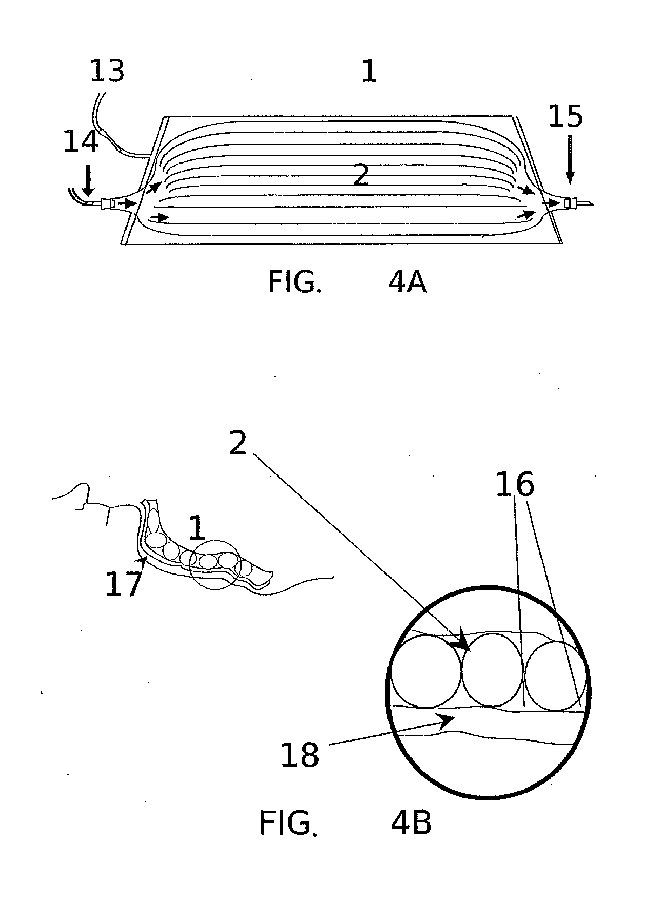

Figure 2 shows in detail a second embodiment of the cervical blanket, when the fluid used is liquid. A) a hollow, plastic, tubular structure is observed; the structure comprising ducts (2) through which the liquid fluid (3) circulates and comprises a fluid inlet (14) that receives the fluid (3) from the heat exchanger (4), and an outlet (15) which transports the fluid (3) to the heat exchanger, wherein the inlet connection means is subdivided into a series of ramifications corresponding to the ducts (2) through which the fluid (3) circulates until reaching the liquid fluid outlet zone where they converge into a single duct which returns the fluid to the heat exchanger. In a more particular embodiment, there exists a vacuum line (13) which enables the cavity between the cervical blanket and patient's skin to be subjected to constant aspiration to cause vacuum. This causes that both walls of this cavity to be in close proximity until no space is left between each other and they become fixed adopting the shape of the contacting surface. B) A cross-sectional view of the cervical blanket is observed (1) over a patient and a zone amplification. Within this view the ducts are shown (2) through which the fluid circulates, a virtual space (16), the zone where vacuum (17) is applied between the internal sheet (18) of the cervical blanket (1) and the patient's skin. The internal structure (tubular) which is deflated, i.e., empty, is rather a septal structure like a bee hive, i.e., each tube continues at the neighbor wall. Once expanded through the fluid, they give the blanket the shape of a crust covering the neck with these structures (2).

Within the context of the present invention, body temperature should be understood as a person's core temperature. This is because the present invention relates to whole body temperature modification and not to localized and restricted temperature limited to a part of the person's body.
The present invention is based on the application of convection principle, i.e., heat transfer through a fluid in motion which is either air or water, but applied to the neck of the patient.
From the anatomical point of view, neck is a favorable site where convection mechanisms for temperature transfer can be applied, either through water or air, because: i) the blood flow to the head is constant and does not comprise the variations of blood flow to the extremities caused by vasoconstriction phenomena; ii) blood vessels that transport blood to the brain, travel superficially through the whole neck, iii) carotid arteries run 1 cm below the skin surface and jugular veins are exposed in the neck surface; iv) the amount of blood pumped per minute from the heart of an adult at rest is 5 liters, 1 liter of which travels to the head; v) the neck skin (dermis and epidermis) is generally thinner than in other anatomic areas such as, for example, abdomen, thorax. If heat or cold is applied through air or water convection to the neck, the temperature of air or water is quickly transferred, first to the skin and then to the neck tissues comprising vessels through which blood travels to the brain and travels back from the brain. If we know that within a minute more than 20% of the blood pumped from the heart passes through the neck to the brain through carotid veins and travels back through jugular veins form brain to heart, within 5 minutes a blood flow of 5 liters will have passed both ways through the zone where the temperature is applied, travelling upstream to the brain through the artery and travelling back through the vein. Consequently, the design of a system of heat application through convection, either through the air or through a liquid fluid confined to the cervical or brain area is effective to transfer temperatures to the patient.
In one embodiment, the present invention corresponds to a noninvasive system for rising or lowering body temperature of a person in a controlled manner, comprising a cervical blanket (1) with ducts (2) through which a fluid circulates (3); a fluid (3) capable of transferring heat from and to external bodies; a heat exchanger (4) which rises or lowers the temperature of the fluid circulating inside the ducts (2) of the cervical blanket (1); connection means (5) between the cervical blanket ducts and the heat exchanger (4); a body temperature sensor (6); and a controller (7) which rises or lowers the fluid temperature according to a predetermined temperature and according to the temperature measured by the body temperature sensor.
In a embodiment, when the heat exchange fluid is a liquid fluid, for example water, the cervical blanket (1) adopts the shape of a hollow, plastic structure, which adapts to the surface under the neck and shoulders of the patient and through circulation of a liquid fluid at different temperatures therein, heat is transferred or heat losses are increased by convection. It can be sterilized to be reused, not disposable. In this case, the cervical blanket (1) is a tubular structure comprising a fluid inlet on the right hand side and an outlet on the left hand side wherein at the portion that is in contact with the patient the single inlet piping is subdivided into a series of ramifications intended to supply the water fluid in a laminar manner like a mesh in order to cover the largest skin surface to converge, at the water outlet zone, into one single duct that returns water, that already transferred heat to a patient, to the heat exchanger. The cervical blanket (1) can be adapted but can not be dilated easily so that even with larger fluid fluxes, it does not become globular in shape. It is completely coated by a plastic coating with the shape of a patient's neck and shoulder surface wherein the external face acts as a thermal insulator (to prevent effectiveness loss and to only allow patient temperature exchange and not room temperature exchange) and the internal face comprises a mechanism for adapting to the patient's neck. To optimize the convection temperature transfer, when a liquid fluid circulates through the ducts (2) of the cervical blanket (1) a perfect contact between surfaces is required and by means of a specific interface, the cervical blanket can be adapted to the patient's neck surface without exerting uneven pressure on different areas and without occluding venous circulation. This interface consists of an air bag located between the surface of the skin and the surface of the blanket lines transporting liquid fluid. This bag comprises a connector that can be adapted to any vacuum systems available in every medical center.
The cervical blanket (1) may have different sizes and shapes so that it can be adapted for use in pediatric or adult patients.
In a further embodiment of the invention, the fluid (3) is a liquid fluid. In a more particular embodiment, the fluid (3) is water. In this case, the heat exchanger (4) corresponds to a portable structure operated by batteries or connected to power supply and comprises a water cooler or heater together with a centrifugal pump which propels liquid fluids; it comprises wheels and a plastic tank for storage, with volumes ranging from 1 to 20 liters. At the rear, the heat exchanger comprises two liquid fluid inlets and two outlets which are pressure connectors for the cervical blanket system (1).
In a further embodiment, when fluid is liquid, the means of connection (5) correspond to 1 to 10 meter lines and comprise an adapter located at the heat exchange (4) liquid fluid outlet and an adapter located at the cervical blanket (1) liquid fluid inlet and outlet. The means of connection (5) corresponding to a pipe wherein liquid fluid is transported are made of plastic, hypoallergenic material which is thermostable at temperatures from 10°C to 43°C.
The controller (7) comprises a control-console wherein a power button is located; a temperature selector to select the temperature at which the fluid will be heated or cooled; a pump flow selector; the flow being expressed for example as liters per minute; and a display that provides information regarding the temperature selected at the temperature selector, the liquid fluid outlet temperature through the heat exchanger outlet duct, the patient's temperature, the rate at which the pump operates, for example, in revolutions per minute, and the fluid flow per minute.
The patient's temperature sensor (6) corresponds to an esophageal thermometer equipped with a long cable which extends from the patient's esophagus and it is connected to the controller (7).

### METHOD FOR RISING OR LOWERING A PATIENT'S TEMPERTURE BY USING THE SYSTEM OF THE PRESENT INVENTION

Once the cervical blanket is installed on the patient (1), a body temperature sensor is inserted (6), for example, an esophageal thermometer connected to the controller (7).

The console is switched on and the patient target temperature is selected. The means of connections (5) are connected from the heat exchanger (4) to the cervical blanket (1) making sure that the cervical blanket (1) fluid inlet does not collapse. In a particular example, when the fluid (3) is liquid, the proper size of the blanket is
chosen. A temperature sensor (6), for example, an esophageal thermometer which is inserted into the patient is connected to the controller (7). The cervical blanket (1) is placed over the patient's neck allowing the blanket to accommodate without exerting pressure. Then, the blanket must be best adapted to the neck by using both hands and when this adaptation is considered appropriate without having neck compressed areas, vacuum is connected to the bag which is in contact with the patient's skin. After maximum vacuum is applied to the bag which is in contact with the patient, it has to be verified that the structure is adapted to the patient's neck, with "memory". In order to optimize temperature transfer, a gel is then applied between the skin and the cervical blanket for maximum contact between them.

The heat exchanger motor is switched on, and the following is selected: i) water temperature, ii) patient's target temperature and iii) fluid flow will be modified according to patient cooling or warming rate.

Unlike most of the effective cooling systems, this is a novel noninvasive patient cooling and warming system and it is more efficient for temperature transfer than the existing water mattresses which only cover patient's thoracic and back surfaces; some surfaces may not be in direct contact and the laminar flow over the skin is not achieved.

### APPLICATION EXAMPLES

In the specialized literature, systems for managing temperature through convection comprise cooling average rates from 2 to 1.5°C an hour (Therapeutic hypothermia and controlled normothermia in the intensive care unit: Practical considerations, side effects, and cooling methods. Crit Care Med 2009 Vol. 37, No. 3) which does not differ from the present invention.

The cooling rate achieved by the system of the present invention is rather higher due to the closeness to neck blood.

In one embodiment of the present invention, when the heat exchange fluid is water, cooling rate is around 2°C or higher per hour.

However, in addition to achieve similar or higher cooling rates compared to those described in the literature for commercially available products, the present invention has the following comparative advantages with regard to other methods, namely:
1. The existing products that modify body temperature cover large areas of the skin, either thorax or the entire head resulting in a difficult daily management of patients. Patients with head trauma or brain damage following cardiac arrest require brain signs monitoring which are detected through elements in contact with the scalp, for example, an electroencephalogram, or elements that penetrate the cranial bone to reach brain structures such as Camino intracranial pressure catheters with Lycox. If the technology for modifying the patient's temperature obstructs the access to the scalp or cranium, this must be removed while monitoring brain sings leading to a discontinuation of the hypothermia therapy. The present invention which is limited to patient's neck and shoulders does not obstruct the access to the patient's mouth, airways or cranium. On the other hand, products for modifying patient's temperature that have to be in contact with patient's torso covering large contact surfaces obstruct the inspection of the thorax and abdomen or the access thereto, as well as important access sites for medical exams such as echocardiograms, electrocardiograms, abdominal echotomography or procedures such as surgeries or punctures.
   These transient interruptions are not devoid of complications, specially, with regard to patient overheating since the initial condition may be worsened.
   Finally, punctures must be done on the neck when admitted to the Intensive Care Unit in order to introduce catheters that remain in situ even two weeks so that once installed and covered by the present invention, they do not have to be removed until two week later, i.e., when hypothermia treatment is no longer required.
2. The present invention is easy to install.
3. The closeness to structures such blood vessels to achieve blood cooling makes the present invention unique. Unlike the present invention which only covers the neck surface, prior art products for heat transfer are less effective since they must cover large areas of the thorax and abdomen skin surfaces located far apart.

## Claims

1. Noninvasive system for rising or lowering a patient's body temperature in a controlled manner, wherein the system comprises:
a. a cervical blanket (1) comprising ducts (2) through which liquid circulates (3);
b. liquid (3) capable of transferring heat from and to external bodies;
c. a heat exchanger (4) which rises or lowers the liquid temperature that circulates through cervical blanket ducts;
d. means of connection (5) between ducts (2) of the cervical blanket (1) an the heat exchanger (4);
e. a body temperature sensor (6);
f. a controller (7) which rises or lowers liquid temperature according to a predetermined temperature and according to the temperature measured by the body temperature sensor (6), wherein the cervical blanket (1) is a hollow, plastic, sterilizable structure, and comprises a tubular structure comprising a liquid inlet (14) and an outlet (15) wherein the internal face of the tubular structure is subdivided into a series of ducts (2), so that the liquid is supplied in a laminar manner, and wherein at the liquid outlet zone such ducts converge into a single duct which returns the liquid to the heat exchanger, and wherein the cervical blanket (1) is completely coated by a plastic coating with the shape of a patient's neck and shoulder surface, wherein the external face of the tubular structure acts as a thermal insulator and the internal face comprises a mechanism (18) for adapting to the patient's neck.

2. Noninvasive system for rising or lowering a patient's body temperature in a controlled manner according to claim 1, wherein the controller (7) comprises a control-console where the power button is located; a temperature selector to select the temperature at which the fluid will be heated or cooled; a pump flow selector; the flow being expressed for example as liters per minute; and a display that provides information regarding the temperature selected at the temperature selector, the liquid fluid outlet temperature through the heat exchanger outlet duct, the patient's temperature, the rate at which the pump operates, for example, in revolutions per minute, and the fluid flow per minute.

3. Noninvasive system for rising or lowering a patient's body temperature in a controlled manner according to claim 1, wherein the patient's temperature sensor (6) is an esophageal thermometer equipped with a long cable which extends from the patient's esophagus and it is connected to the controller (7).

4. Noninvasive system for rising or lowering a patient's body temperature in a controlled manner according to claim 1, wherein the fluid (3) is water.

5. Noninvasive system for rising or lowering a patient's body temperature in a controlled manner according to claim 1, wherein the means of connection (5) correspond to 1 to 10 meter lines and comprise an adaptor located at the heat exchange (4) liquid fluid outlet and an adaptor located at the cervical blanket (1) liquid fluid outlet and inlet, and wherein the means of connection (5) comprise a pipe wherein fluid (3) is transported, and wherein they are made of plastic, hypoallergenic, and temperature stable material at temperatures ranging from 10°C to 43°C.

6. Noninvasive system for rising or lowering a patient's body temperature in a controlled manner according to claim 1, wherein the heat exchanger (4) is a portable structure operated by batteries or connected to power supply and comprises a liquid fluid heater and cooler, together with a centrifugal pump for propelling liquid fluids, and it comprises wheels and a plastic tank for storage, and wherein at the rear face it comprises two liquid fluid inlets and two outlets which are pressure connectors for the cervical blanket system (1).

## Patentansprüche

1. Nicht invasives System zur kontrollierten Erhöhung oder Senkung der Körpertemperatur eines Patienten, worin das System folgendes umfasst:
a. eine Zervikaldecke (1) mit Leitungen (2) durch die ein Fluid (3) fliesst;
b. ein Fluid (3), welches Wärme auf und von externen Körpern übertragen kann;
c. einen Wärmeübertrager (4), der die Temperatur der Flüssigkeit in den Leitungen der Zervikaldecke erhöht oder senkt;
d. Verbindungen (5) zwischen den Leitungen (2) der Zervikaldecke (1) und dem Wärmeübertrager (4);
e. einen Sensor der Körpertemperatur (6);
f. einen Regler (7), der die Temperatur des Fluids in Abhängigkeit der eingestellten Temperatur und in Abhängigkeit der vom Sensor für Körpertemperatur (6) gemessenen Temperatur erhöht oder senkt, bei dem die Zervikaldecke (1) eine hohle, sterilisierbare Kunststoffstruktur ist, die eine rohrförmige Struktur mit einem Eingang (14) und einem Ausgang (15) für die Flüssigkeit umfasst, und bei der die innere Seite der rohrförmigen Struktur sich in eine Anzahl von Leitungen (2) verzweigt, so dass die Flüssigkeit schichtartig verteilt wird und bei dem sich die erwähnten Leitungen im Bereich des Austritts der Flüssigkeit zu einer einzigen Leitung vereinen, welche die Flüssigkeit zum Wärmeübertrager zurückführt und bei dem die Zervikaldecke (1) vollständig von einer Kunststoffplane bedeckt ist, die die Form der Hals- und Schulteroberfläche des Patienten hat, deren äussere Schicht zur thermischen Isolierung dient und deren innere Schicht einen Mechanismus (18) hat, der sich dem Hals des Patienten anpasst.

2. Nicht invasives System zur kontrollierten Erhöhung oder Senkung der Körpertemperatur eines Patienten gemäss Anspruch 1, worin der Regler (7) zur Kontrolle eine Konsole mit einem Einschaltknopf hat, sowie einen Temperaturwähler um die Temperatur einzustellen, auf die das Fluid erwärmt oder gekühlt werden soll; einen Wähler für den Pumpenfluss, ausgedrückt in Liter pro Minute, zum Beispiel, und einen Bildschirm, auf dem die Information bezüglich der auf dem Temperaturwähler gewählten Temperatur, der Ausgangstemperatur des Fluids in der Ausgangsleitung des Wärmeübertragers, der Temperatur des Patienten, der Geschwindigkeit der Pumpe, zum Beispiel in Umdrehungen pro Minute, und dem Fluidfluss pro Minute angezeigt wird.

3. Nicht invasives System zur kontrollierten Erhöhung oder Senkung der Körpertemperatur eines Patienten gemäss Anspruch 1, worin der Sensor der Temperatur des Patienten (6) ein Speiseröhrenthermometer ist, welches ein langes Kabel hat, das von der Speiseröhre des Patienen zum Regler (7) führt.

4. Nicht invasives System zur kontrollierten Erhöhung oder Senkung der Körpertemperatur eines Patienten gemäss Anspruch 1, worin das Fluid (3) Wasser ist.

5. Nicht invasives System zur kontrollierten Erhöhung oder Senkung der Körpertemperatur eines Patienten gemäss Anspruch 1, worin die Verbindungen (5) Schläuche mit einer Länge von 1 bis 10 Metern sind und über einen Adapter verfügen, der sich am Austritt des flüssigen Fluids aus dem Wärmeübertrager (4) befindet, und über einen Adapter am Eingang und Austritt des flüssigen Fluids aus der Zervikaldecke (1), und dass die Verbindungen (5) in einer Leitung bestehen, welche das flüssige Fluid (3) befördert und aus Kunststoff, hypoallergen und innerhalb einer Temperaturspanne von 10° C bis 43°C stabil sind.

6. Nicht invasives System zur kontrollierten Erhöhung oder Senkung der Körpertemperatur eines Patienten gemäss Anspruch 1, worin der Wärmeübertrager (4) eine tragbare Struktur ist, welche batteriebetrieben ist oder an das Stromnetzt angeschlossen werden kann und ein Gerät zum Erwärmen oder Kühlen von Wasser besitzt, sowie eine Zentrifugalpumpe, welche das flüssige Fluid in Bewegung bringt, sowie über Räder und einen Kunststoffbehälter für das Fluid verfügt, und auf seiner Rückseite zwei Ausgänge und zwei Eingänge für flüssiges Fluid hat, welche Druckverbindungen für das System der Zervikaldecke haben (1).

## Revendications

1. Système non invasif pour augmenter ou diminuer la température corporelle d'un patient de façon contrôlée, en quoi le système comprend :
a. une couverture cervicale (1) avec des conduits (2) dans lesquels circule un fluide (3);
b. un fluide (3) capable de transférer de la chaleur depuis et vers des corps externes;
c. un échangeur de chaleur (4) qui augmente ou diminue la température du liquide qui circule dans les conduits de la couverture cervicale;
d. des connecteurs (5) entre les conduits (2) de la couverture cervicale (1) et l'échangeur de chaleur (4);
e. un senseur de température corporelle (6);
f. un contrôleur (7) qui augmente ou diminue la température du fluide en fonction d'une température prédéterminée et en fonction de la température mesurée par le senseur de température corporelle (6), où la couverture cervicale (1) est une structure creuse, plastique, pouvant être stérilisée, qui comprend une structure tubulaire ayant une entrée (14) et une sortie (15) de liquide, et où la face interne de la structure tubulaire se divise en une série de conduits (2) de façon telle que le liquide est fourni de façon laminaire et que, dans la zone de sortie du liquide, ces tubes convergent en un seul conduit qui retourne le liquide à l'échangeur thermique et où la couverture cervicale (1) est totalement recouverte par une enveloppe plastique ayant la forme de la surface du cou et des épaules du patient, où la face externe agit comme isolant thermique et la face interne a un mécanisme (18) pour s'adapter au cou du patient.

2. Système non invasif pour augmenter ou diminuer la température corporelle d'un patient de façon contrôlée, conformément à la revendication 1, en quoi le contrôleur (7) comprend une console de contrôle sur laquelle il y a un bouton d'allumage; un commutateur de température pour choisir la température à laquelle on va chauffer ou refroidir le fluide; un commutateur de flux de la pompe exprimé, par exemple, en litres par minute; et un écran où s'affiche l'information relative à la température choisie dans le commutateur de température, la température de sortie du fluide par le conduit de sortie de l'échangeur de chaleur, la température du patient, la vitesse de fonctionnement de la pompe, en tours par minute par exemple et le flux de fluide par minute.

3. Système non invasif pour augmenter ou diminuer la température corporelle d'un patient de façon contrôlée, conformément à la revendication 1, en quoi le senseur de température du patient (6) correspond à un thermomètre oesophagique, qui comprend un long câble qui part de l'oesophage du patient et est connecté au contrôleur (7).

4. Système non invasif pour augmenter ou diminuer la température corporelle d'un patient de façon contrôlée, conformément à la revendication 1, en quoi le fluide est de l'eau (3)

5. Système non invasif pour augmenter ou diminuer la température corporelle d'un patient de façon contrôlée, conformément à la revendication 1, en quoi les connecteurs (5) correspondent à des lignes de 1 à 10 mètres et ils disposent d'un adaptateur qui se trouve à la sortie de fluide liquide de l'échangeur de chaleur (4)et un adaptateur qui se trouve à l'entrée et la sortie de fluide liquide de la couverture cervicale (1), et où les connecteurs (5) comprennent un conduit qui transporte le fluide (3) et ils sont en matériau plastique, hypoallergénique et stable à la température dans des amplitudes de température pouvant aller de 10°C à 43 °C.

6. Système non invasif pour augmenter ou diminuer la température corporelle d'un patient de façon contrôlée, conformément à la revendication 1, en quoi l'échangeur de chaleur (4) est une structure portable, qui fonctionne avec des batteries ou branché au réseau électrique et qui possède un chauffage et un refroidisseur de fluides liquides, ainsi qu'une pompe centrifuge qui propulse le fluide liquide, sur roulettes et un bac plastique pour entreposer, et qui sur sa face postérieure a deux sorties et deux entrées de fluide liquide qui sont des connecteurs à pression pour le système de la couverture cervicale (1).
